# EUROPEAN PATENT APPLICATION

(11) **EP 4 335 464 A1**
(43) Date of publication of application: **13.03.2024**
(21) Application number: 23191915.0
(22) Date of filing: 17.08.2023
(51) Int. Cl.: A61L 2/10, A61L 2/24, G06F 3/0354, G06F 3/039, A61L 2/18, G06F 1/16

(54) **AN ADJUSTABLE SANITIZING APPARATUS FOR A TOUCH SCREEN DEVICE**

(30) Priority: 08.09.2022 FI 20225781
(71) Applicant: Mr-Visualize Oy, 01530 Vantaa (FI)
(72) Inventor: MALINEN, Reino, 01530 Vantaa (FI)
(74) Representative: Boco IP Oy Ab

(57) **Abstract**

The present invention relates to a sanitizing apparatus for providing a sanitized user interface for a touch screen unit. The sanitizing apparatus comprising a film conveyor apparatus, a housing, a film, a control unit and a sanitizing unit. The film conveyor apparatus is configured to move a sanitized portion of the film in front of a touch surface of the touch screen unit. The sanitizing apparatus sanitizes a portion of the film by means of UVC light. The film conveyor apparatus comprises a chassis, and a plurality of rolls attached to the chassis. The plurality of rolls comprises a plurality of deflector rolls configured to guide the film about the touch screen unit, and a drive roll configured to drive the film. The plurality of rolls is arranged as two groups of rolls. The chassis comprises two pairs of deflector roll support means configured to be assembled on two opposite sides of the touch screen unit. Each pair of deflector roll support means rotatably supports all rolls of one of the two groups of rolls.

## Description

### Field

The present invention relates to an apparatus related to sanitizing user interfaces. More particularly, the invention relates to and apparatus that provides a sanitized protection film in front of a touch screen surface.

### Background

Recent pandemic situation has increased need to make public spaces and services safer to avoid spreading of contagious diseases. Any surface that needs to be touched by several members of the public causes a risk of spreading bacteria and/or viruses that cause contagious diseases. Touch screen devices are commonly used broadly for providing services for public, for example to provide information on where to find a wanted service in a mall, or to order food in a fast-food restaurant. Tens or hundreds of people may touch the same screen during a single day. Several solutions have been developed to improve microbiological safety of surfaces to be touched.

One line of solutions relies on using a transparent film on or in front of the touch display, such that a clean portion of the transparent film can be provided to the user to touch. Film can either be discarded after use or sanitized.

Short-wave ultraviolet radiation also known as ultraviolet C radiation or UVC light has been found to be an effective way of sanitizing surfaces. UVC light is, however, harmful to humans, and should only be applied on the surface when no human is exposed to it. UVC light is applied to sanitize touchable surfaces, such as transparent films used in this type of protective devices.

### Description of the related art

Japanese patent application JP2022025880 A discloses a display device with a movable surface formed by a transparent film, that is sterilized by ultraviolet rays. Patent application WO2019/206802 A1 discloses a medical treatment device with a foil in front of its input surface, such as touch screen or keyboard, wherein the device disinfects the foil using UV light or using liquid film.

Patent application US20120268394 A1 discloses a system and method for sanitizing touch screen of electronic devices with two motor rolls positioned on opposite sides of the touch screen.

Finnish utility model FI 12997 Y1 discloses a touch screen covered with a rotatable, transparent film that is being sanitized by a sanitizing unit.

### Summary

An object is to further improve sanitizing apparatus that provides a protective film used as touch surface for a touch screen device. The objects of the present invention are achieved with an apparatus according to the characterizing portion of claim 1.

The preferred embodiments of the invention are disclosed in the dependent claims.

The present invention is based on the idea of providing a sanitizing apparatus having a film conveying apparatus with an easily adjustable, modular type of chassis.

According to a first aspect, a sanitizing apparatus providing sanitized user interface for a touch screen unit is provided. The sanitizing apparatus comprises a film conveyor apparatus, a housing, a film, a control unit and a sanitizing unit. The film conveyor apparatus is configured to move a sanitized portion of the film in front of a touch surface of the touch screen unit. The sanitizing apparatus comprises a sanitizing unit configured to sanitize a portion of the film by means of UVC light after the portion of the film has been used for user interaction. The portion of the film to be sanitized is enclosed inside the housing during said sanitization. The film conveyor apparatus comprises a chassis, and a plurality of rolls attached to the chassis. The plurality of rolls comprise a plurality of deflector rolls configured to guide the film about the touch screen unit, and a drive roll configured to drive the film.

The plurality of rolls is arranged in two groups of rolls. The chassis comprises two pairs of deflector roll support means configured to be assembled on two opposite sides of the touch screen unit by attaching the deflector roll support means to the touch screen unit. Each pair of deflector roll support means is configured to rotatably support all rolls of one of the two groups of rolls.

According to some aspects, each deflector roll support means comprises a bracket and a bearing housing means. The bracket is configured for fitting and removably attaching the deflector support means to a desired position of the touch screen unit. The bearing housing means is configured to house bearings that enable rotation of rolls and to determine position of rolls supported thereby with respect to the touch screen unit when assembled.

According to some aspects, bearing housing means of each pair of deflector support means are mutually parallel and installation holes configured to receive bearings and axes of rolls in the two parallel bearing housing means are mutually mirrored.

According to some aspects, at least two of the bearing housing means are configured to be slidably coupled to the respective bracket. The bearing housing means is configured to be slidable with respect to the respective bracket only in direction parallel to the longitudinal axis of rolls supported thereby. The bearing housing means is configured to be removably fixed to the bearing housing means by means of coupling of the rolls to the bearing housing means.

According to some aspects, one deflector roll in both groups of rolls is configured to operate as gap determining deflector roll, wherein these two gap determining deflector rolls are disposed on two opposite sides of the touch surface of the touch screen device, and wherein said gap determining deflector rolls are configured to determine and maintain a predetermined distance of the portion of the film in front of the touch surface when the touch screen is not used for user input during a user interaction.

According to some aspects, the predetermined distance is between 0.3 and 0.6 mm from the touch surface.

According to some aspects, the film is a transparent FEP film, PFA film or ETFE film, and thickness of the film is in the range of 0.2 to 0.3 mm.

According to some aspects, bearing housing means of each pair of deflector roll support means comprise mutually mirrored arrangement of installation holes configured to house bearings and rolls.

According to some aspects, at least one of the deflector rolls is an adjustable deflector roll. The position of the adjustable deflector roll is configured to adjust tension of the film.

According to some aspects, bearing housing means of at least one pair of deflector roll support means comprise elongated installation holes for adjustably supporting the adjustable deflector roll.

According to some aspects, outer surface of the drive roll is coated with a friction increasing coating.

According to some aspects, outer surface of a deflector roll is coated with a friction increasing coating, and wherein the deflector roll coated with the friction increasing coating is operationally coupled with the drive roll for applying drive action on the film at both sides of the touch screen unit.

According to some aspects, the sanitizing apparatus comprises at least one of a pre-cleaning scraper and a wet cleaning roll. The pre-cleaning scraper is configured to drag off scrap particles from the film. Outer surface of the wet cleaning roll is covered by a felt. The felt is configured to be wetted with a wet cleaning substance. The wet cleaning roll is configured to wash away scrap particles, grease and various chemical substances from the film.

According to some aspects, the at least one adjustable deflector roll is further configured to operate as the wet cleaning roll.

According to some aspects, the wet cleaning roll is configured to rotate in opposite direction in comparison to the movement of the film.

According to some aspects, outer surface of a deflector roll is coated with a friction increasing coating. The deflector roll coated with the friction increasing coating is operationally coupled with the wet cleaning roll for rotating the wet cleaning roll.

According to some aspects, the film sanitizing unit is configured to expose the film to UVC light after pre-cleaning step performed by at least one of the pre-cleaning scraper and the wet cleaning roll.

According to another aspect, a touch screen apparatus comprising a touch screen unit and the sanitizing apparatus according to any of the above aspects is provided.

The present invention has the advantage that the same sanitizing apparatus can be easily adjusted to fit different sizes of touch screen devices, and the sanitizing apparatus is also easy to assemble and service.

### Brief description of the drawings

In the following the invention will be described in greater detail, in connection with preferred embodiments, with reference to the attached drawings, in which
Figure 1 is an isometric perspective drawing of a touch screen apparatus
Figure 2 illustrates assembling of the touch screen apparatus
Figure 3 illustrates structure of the film conveying apparatus
Figure 4 is a partially split drawing of the structure of the film conveying apparatus
Figures 5A and 5B illustrate a cross-section of the touch screen apparatus
Figures 6A and 6B illustrate film conveying apparatus equipped with pre-cleaning means
Figures 7A to 7D illustrate film conveying apparatus equipped with pre-cleaning means
Figure 8 illustrates schematically control arrangement of the film sanitizing apparatus

### Detailed description

In the following, the relative term in front refers to a spatial indication which relates to orientation of a component concerned during its normal use.

In normal use, a touch screen unit has a typically flat surface that operates as a touch sensitive display. A film in front of a touch surface refers to a relative position of the film with respect to the touch surface of a touch screen device such that the film preferably fully covers the operating surface of the touch screen.

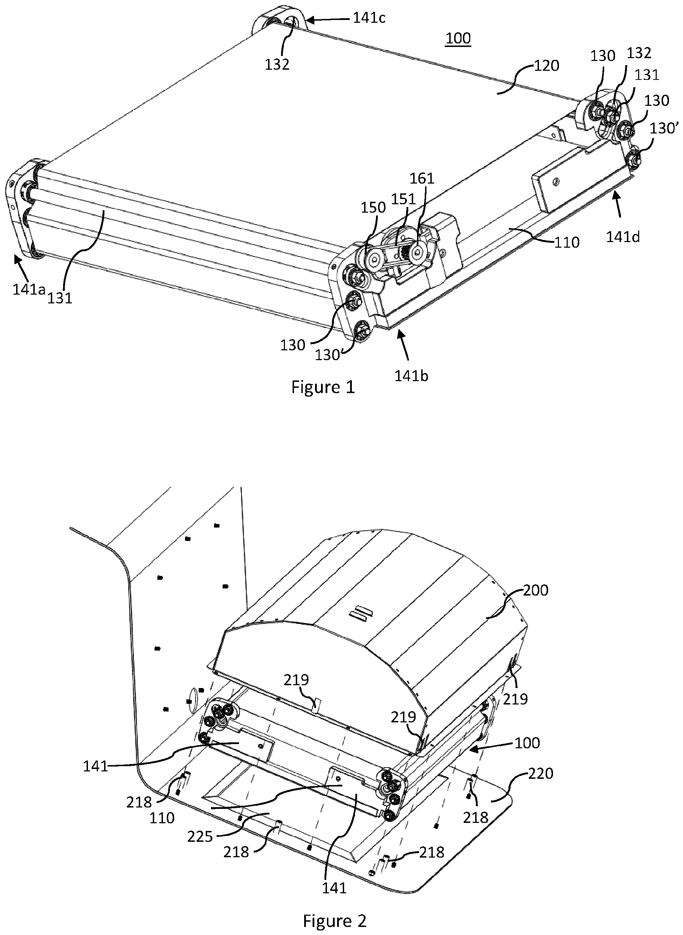

The figure 1 shows an isometric perspective drawing of a touch screen apparatus (100) according to embodiment of the invention.

A touch screen apparatus (100) comprises a touch screen unit (110) with a touch surface (not shown) operable by a user and a sanitizing apparatus. In the figure 1, the touch surface is directed downwards to enable displaying structural and operational elements of the touch screen apparatus. The touch screen unit is preferably of capacitive type, which has been tested and proofed to be well functioning with protective film between the touch surface and the user's hand.

The sanitizing apparatus comprises a film (120). For best results, the film (120) is transparent such that the user can easily see all data displayed on the touch screen. The film is preferably configured as an endless loop that circulates about the touch screen unit (110), guided by a film conveying apparatus. The loop may be manufactured for example by attaching together two opposite ends of a film cut in wanted width and length. The film (120) is conveyed about the touch screen unit (110) by a film conveyor apparatus that comprised in the sanitizing apparatus.

The film conveyor apparatus preferably comprises a plurality of mutually separate structural parts, herein called as deflector roll supports (141). Deflector roll supports (141) support a plurality of deflector rolls (130, 130', 131) and a drive roll (150). The plurality of deflector roll supports (141) is herein referred to as a chassis.

Conveying of the film by the film conveyor apparatus is controlled by a control unit (not shown), which may be attached for example to one of the deflector roll supports (141) or to the touch screen unit (110). The film (120) is being moved intermittently by the film conveyor apparatus under control of the control unit (not shown). The film conveyor apparatus comprises a plurality of deflector rolls (130, 130', 131) and a drive roll (150), which are together configured to convey the film (120) about the touch screen unit (110). Deflector rolls (130, 130', 131) and the drive roll (150) are attached between two deflector roll supports (141) assembled in wanted positions with respect to the touch screen unit (110). The modular structure enables the chassis and the entire film conveyor apparatus to be easily adjustable to touch screen units (110) of different sizes. The chassis shown in the figure 1 comprises four deflector roll supports (141), which are attached to the touch screen unit (110), each one approximately at a corner of the rectangular touch screen unit (110). Deflector rolls (130, 130', 131) and the drive roll (150) are all attached to the deflector roll supports (141). Bearings, such as ring bearings, are provided at both ends of each of the deflector rolls (130, 130', 131) and the drive roll (150) to enable smooth rotation thereof about their rotation axes. Hereinafter, the plurality of reflector rolls (130, 130', 131) and drive roll (150) can be jointly referred as rolls.

By means of the deflector roll supports (141), film guiding deflector rolls (130, 130', 131) are preferably positioned in two groups on two opposite sides of the touch screen unit (110) as shown in the figure 1. Preferably, the chassis comprises two pairs of deflector roll supports (141a, 141b; 141c, 141d) attached on the two opposite sides of the touch screen unit (110). Each pair of deflector roll supports (141a, 141b; 141c, 141d) is arranged in parallel and comprise mutually mirrored installation openings configured to receive bearings and applicable one of the plurality of deflector rolls (130, 130', 131) or the drive roll (150). Preferably, deflector roll supports (141) are mutually mechanically separate parts, designed to be fastened to a wanted location of the touch screen unit (110) with fastening means, such as screws or bolts. Mutually mechanically separate deflector roll supports (141), which are relatively small in size, can easily be manufactured for example by 3D printing.

According to some exemplary alternative implementations, deflector roll supports (141a, 141b; 141c, 141d) and/or brackets (142) of deflector roll supports (141a, 141b; 141c, 141d) supporting one group of rolls at one side of the apparatus may form a single structural portion of the chassis. According to other alternative implementation, deflector roll supports (141a, 141b; 141c, 141d) and/or brackets (142) at each side of the apparatus supporting different groups of rolls may form a single structural portion of the chassis. Mechanically combined deflector roll supports and/or brackets, however, makes the chassis to be less adjustable, and sets requirements for manufacturing equipment due to bigger size of the parts in comparison to mechanically separate deflector roll supports (141), which are connected to each other only indirectly by the rolls and the touch screen unit to which they are removably attached.

The film (120) is driven by a drive roll (150) that is rotated by drive means (350) such as a motor, preferably an electrical motor. In the shown embodiment, a drive wheel (161) attached to a rotating axis of the drive means (350) is coupled to the axis of the drive roll (150) by a drive belt (151), but any known alternative for coupling drive force to the drive roll (150) is applicable. The drive means (350) is preferably attached to one of the deflector roll supports (141b) of the chassis to facilitate ease of assembling of the film conveying apparatus on the touch screen unit (110). Alternatively, the drive means (350) may be attached to the touch screen unit (110) by suitable fastening means. Operation of the drive means (350) is controlled a control apparatus (not shown). According to some embodiments, the drive means (350) is axially coupled to the drive roll (150).

Outer surface of the drive roll (150) interacting with the film (120) is preferably coated with a friction increasing coating, such as natural or synthetic rubber coating or other suitable elastomer coating. As a non-limiting example, during prototype testing, commercially available rubber coating known as Maston RUBBERcomp^{®} was used as friction increasing coating. Sufficient friction of the drive roll (150) is needed for ensuring reliable contact of the drive roll (150) for conveying of the film (120). A benefit of implementing conveying of the film (120) with a single drive roll (150) is that there is no need to ensure full synchronization of a plurality of drive means and/or drive rolls. Having more than one drive rolls may be problematic, because just a slight difference in operating times or rotation rates of two or more drive rolls would significantly increase risk of deteriorating the film.

Friction increasing coating may also be used on at least one further deflector roll (130). Proper drive action of the film (120) can be facilitated by further setting the film (120) with a proper tension. For setting the tension of the film (120), at least one of the deflector rolls is configured as an adjustable deflector roll (131) with adjustable position. Main purpose of the adjustable deflector roll (131) is to enable adjusting tension of the film (120). Adjustment of the position of the adjustable deflector roll (131) may be implemented for example by providing at least one pair of deflector roll supports (141) of the chassis with symmetrically positioned elongate installation openings (132), one elongate installation opening (132) at each side of the film (120), wherein an adjustment means, such as a screw is used to determine position of the end of the adjustable deflector roll (131) within the respective elongate installation opening (132).

Any slipping of film (120) on the drive roll (150) may cause the friction increasing coating thereof to become eventually damaged. This can be avoided by adding another, indirect "driving roll". This can be implemented by coating at least one deflector roll (130) with the friction increasing coating and coupling it operationally to the drive roll (150). This way, slipping of the film (120) can be reduced in particular during acceleration phase of conveying operation. The friction increasing coating on the deflector roll (130) may be similar to that applied on the drive roll (150). The operational coupling between the drive roll (150) and the deflector roll provided with friction increasing coating may be implemented for example by means of a toothed belt (not shown), also known as a cog belt or timing belt. Preferably, the operational coupling between the drive roll (150) and the deflector roll (130) is implemented at the end of these two rolls that is opposite to the end of drive roll (150) that is coupled to the drive means. All deflector roll and drive roll assemblies are provided with bearings that facilitate rotation of the respective deflector roll (130, 130', 131) or drive roll (150) about its rotation axis. Deflector rolls may be assembled using any suitable fastening means. For example, nuts may be used that directly attach to threaded extensions aligned with the rotation axis of the deflector roll.

The drive roll (150), deflector rolls (130, 130'), as well as the adjustable deflector roll (131) preferably have a body made of steel, which is stiff enough for ensuring that the roll does not bend over time due to tension caused by the film (120). By ensuring that all rolls maintain their original shape, even tension of the film (120) can be ensured so that uneven stretching and/or unwanted folding of the film (120) can be avoided. In tests, thin steel-made rolls with diameter between 6 and 10 mm, preferably 8 mm has been found to provide good performance at least up to 350 mm length of the rolls. Deflection and drive rolls with this small diameter have been found to perform particularly well for tensioning the film (120), whereas use of large rolls make proper adjustment of tension of the film less accurate.

The figure 2 illustrates assembling of the touch screen apparatus (100). The touch screen apparatus (100) is preferably covered by a housing. The housing preferably comprises a back cover (200) and a frame (220), which may be comprised in or attached to a stand which is designed for placing of the touch screen assembly on floor. Alternatively, the housing may be designed for placing the touch screen on a wall or hanging it from a ceiling or installing in a rack. The frame (220) has an opening (225) for making the touch surface of the touch screen unit (110) available for users when the touch screen apparatus is assembled between the frame (220) and the back cover (200). The frame (220) and the back cover (200) that form the housing together protect all other structural and functional parts of the touch screen apparatus (100) from the surrounding environment except the touch surface itself and the portion of the film (120) that covers the touch screen. Thus, the touch screen apparatus (100) is safe for use even in public spaces.

For facilitating easy and fast assembly, the back cover (200) may be designed to fit with the chassis such that the touch screen apparatus (100) can be assembled to the frame (220) just by placing the touch screen apparatus (100) in correct place on the frame (220), covering it with the back cover (200) and tightening a plurality of fastening means, such as screws, for fixing the back cover (200) to the frame (220). Shape of the deflector roll supports (141) of the chassis may be designed such that the chassis fits precisely with the back cover (200) and thus helps in positioning of the back cover (200) correctly. Furthermore, the frame (220) may be provided with guide elements (218) projecting towards the back cover (200), which has guide slots or depressions (219) into which the guide elements (218) fit. Guide elements (218) and guide slots and depressions (219) facilitate ease of assembly.

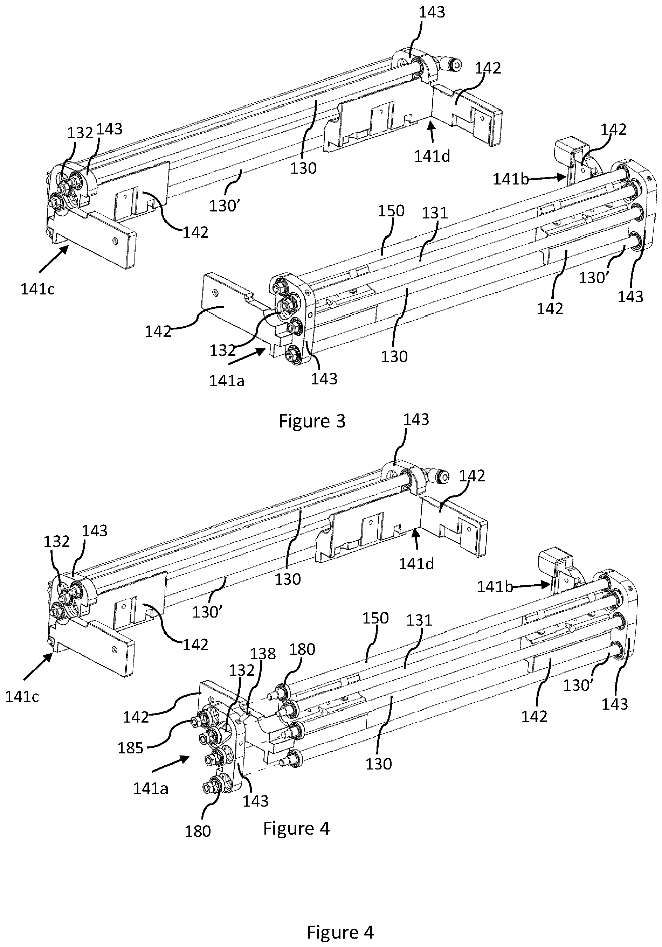

The figure 3 illustrates structure of the film conveying apparatus, showing the chassis and rolls according to some embodiments of the invention. The film (120) has been omitted to give a clearer view of these parts. The chassis comprises four deflector roll supports (141) for rotatably supporting deflector rolls (130, 130', 131) and the drive roll (150). Each deflector roll support (141) comprises a bracket (142) designed and configured for fastening the deflector roll support (141) to the touch screen unit (110) at a wanted position. Each deflector roll support (141) also comprises a bearing housing means (143), which comprises a plurality of installation openings configured to house bearings and thereby also to determine position of the respective deflector roll (130, 130', 131) or the drive roll (150) when assembled. When readily assembled for use, the bearing housing means (143) of the pair of deflector roll supports at each side of the touch screen unit (110) are parallel, and installation openings thereof are mutually mirrored such that all rolls attached to the pair of deflector roll supports (141) are parallel to each other when carried by the pair of deflector roll supports (141). In the shown example, bracket (142) of one of the deflector roll supports (141b) has a specific design to facilitate fastening drive means for the drive roll (150). In the shown design, each bracket (142) forms a straight angle to fit the respective corner of the touch display unit (not shown).

Deflector roll supports (141) are preferably manufactured from suitable photopolymer plastic material by means of 3D-printing. Use of 3D printing enables economical manufacturing of relatively small amounts of deflector roll supports (141) specifically designed to various touch screen models. Also multipart design of the chassis facilitates ease of 3D printing, because deflector roll supports (141) are smaller than if the chassis comprised just one or two parts.

The figure 4 is a partially split drawing of the same parts of the film conveying apparatus as in the figure 3, with the bearing housing means (143) of one of the deflector roll supports (141a) split to show construction thereof in more detail. In this embodiment, rolls are held in place by bolts (185), which are fastened at the threaded ends of rolls, but any alternative fastening means are applicable. When the bearing housing means (143) comprises an elongated opening (132) for assembling the adjustable deflector roll (131), position of the roll and the bearings may be adjusted using one or more adjustment screws (138).

In some embodiments, especially applicable with larger touch screen devices, deflection rolls may need further support to avoid bending thereof due to tension of the film. Such additional support may be achieved by providing one or more intermediate deflector roll supports disposed between two deflector roll supports (141) supporting two opposite ends of rolls (130, 130', 131, 150). Bearing housing means and bearings in such intermediate deflector roll supports should not circumvent the supported roll. For providing additional support to prevent bending of a roll, is sufficient to provide rotational support for the roll in a sector of the circumference of the roll that does not interact with the film (120).

The partially split drawing shown in the figure 4 illustrates a preferable feature of some of the bearing housing means (143) that further facilitates ease of assembly and maintenance of the film conveying apparatus. At least two of the deflector roll supports (141a, 141c) are preferably constructed such that bearing housing means (143) is removably attached thereto. The bearing housing means (143) and the respective bracket (142) may be designed such that the bearing housing means (143) is designed to be removably joined with the bracket (142) by sliding the bearing housing (143) in direction parallel to the longitudinal axes of rolls being supported thereby. Such sliding joint can be achieved by applying well-known sliding dovetail joint principle, which is widely used for example in woodworking. The sliding joint should be designed such that it only allows relative movement of the bearing housing means (143) and the bracket (142) in direction parallel to the longitudinal axes of rolls (130, 130', 131, 150) but disables relative movement of the bearing housing means (143) any other direction. Upon assembling, the bearing housing means (143) is first slidably joined with the bracket (142) and locked into correct position by the fastening means, such as bolts (185).

This kind of removable assembly of the bearing housings (143) on at least one side of the display enables for example changing a damaged or stretched film (120) to a new one without need to disassemble the entire film conveying apparatus. Such common maintenance operation of the film conveying apparatus can be quickly and easily performed on-site, with no need to transport the apparatus to a service center. In the disclosed exemplary embodiment, bearing housings on one side, associated with bearing roll supports 141b and 141d are integral part thereof, while the bearing housings (143) on the other side, associated with deflector roll supports 141a and 141c are provided with slidable joints with the respective bracket (142).

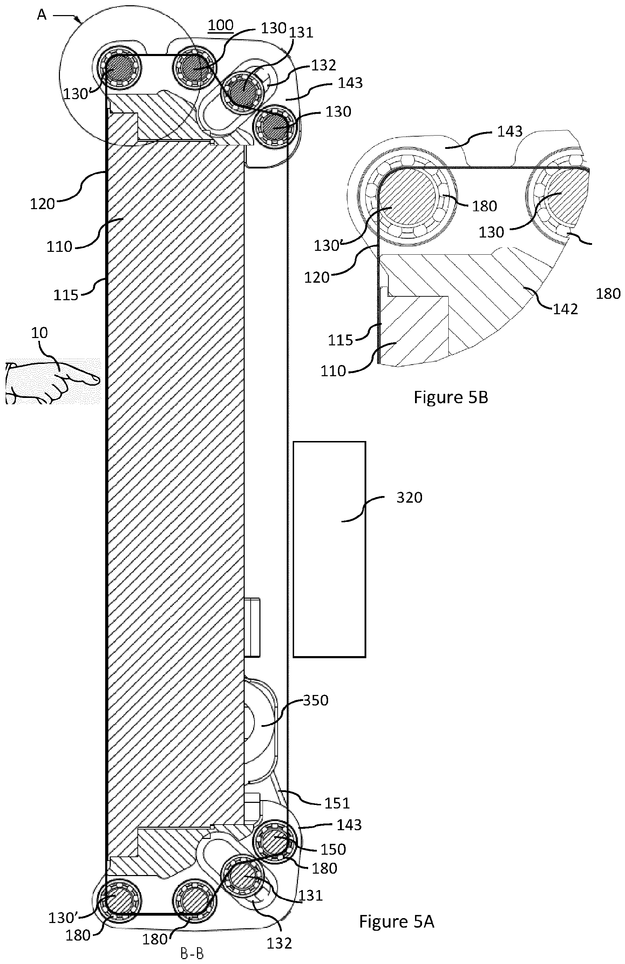

The figure 5A illustrates a cross-section of the touch screen apparatus (100). The figure 5B is an enlarged view of the portion "A" encircled in the figure 5A. The figure is not in scale; in particular, thickness of the film (120) has been exaggerated for visualization. In the following, we use term touch surface (115) to refer to the actual surface used for operating the touch screen unit (110).

A user (10) operates the touch surface (115) of the touch screen unit (110) at the front side of the touch surface (115). The touch screen apparatus comprises a sanitizing unit (320) that is configured to sanitize a portion of film (120) after the film has been used for protecting the touch screen during a user interaction. The sanitizing unit (320) may be provided at the back side of the touch screen unit (110), such that when the touch screen apparatus (100) is properly assembled for use, the sanitizing unit (320) is enclosed inside the housing such that any used, dirty portion of the film (120) that was moved away from reach of the user and is to be sanitized or is being sanitized resides within the housing.

The sanitizing unit (320) applies ultraviolet light for sanitizing at least the film. In some embodiments, the sanitizing unit (320) is attached at the back of the touch screen unit (110). Alternatively, the sanitizing unit (320) may be attached to the back cover (200) or to one or more deflector roll supports (141) the chassis. The location of the sanitizing unit (320) may be rather freely selected, but it should be ensured that the UVC light radiated by the sanitizing unit remains within the space defined by the housing between the touch screen unit (110) and the back cover (200).

This cross-section view shows bearings (180) that enable rotation of each of the deflector rolls (130, 130', 131) and the drive roll (150) about its respective rotation axis. Preferably, bearings (180) are ring bearings, and each installation opening in each bearing housing means (143) is designed to receive a respective bearing or bearings. The drive means (350) may rotate the drive roll (150) via a drive belt (151), for example a toothed belt. Alternatively, any other alternative arrangement for conveying sufficient driving force to the drive roll (150) is applicable. As shown in the figure 5A, on or more adjustable deflector rolls (131) and respective elongate openings (132) may be provided for adjustment of tension of the film (120). In this example, two adjustable deflector rolls (131) are supported by bearing housing means (143), one by each pair of deflection roll supports (141). A cross-section of the bracket (142) is shown in the figure 5A. The shape of the bracket (142) is preferably designed to fit the outer surface of the touch screen unit (110), such that the position of the bracket (142) with respect to the body of the touch screen unit (110) is precise when assembled. A precise position of the assembled bracket (142) facilitates precise position of gap determining deflector rolls (130'), to be discussed below, such that the wanted small gap between the touch surface (115) and the film (120) in front of the touch surface (115) is achieved and maintained.

Two of the deflector rolls have an additional, special role of guiding the film (120) over the touch surface such that a small gap between the film (120) and touch surface is maintained. These deflector rolls with the specific role for determining width of the gap, in other words distance of the film (120) from the touch surface are called herein as distance determining deflector rolls (130'). By maintaining the small gap between the film (120) and the touch surface, friction-caused wear of both the touch surface (115) and the film (120) can be avoided especially when the film (120) is conveyed, while useability of the touch screen remains good. A distance between 0.3 mm and 0.6 mm has been found to give best user experience: the user does not experience an uncomfortable soft or "bending" feeling when operating the touch screen by with the film (120) in between his/her finger and the touch surface (115). Also touch response of the touch screen covered with such 0.3 mm to 0.6 mm film has been found to be at least close to that of a touch screen with no film (120) in front thereof, because a movement of the film by such short distance due to pressing a finger against the film (120) is not easily detectable by the user, and the touch surface is able to reliably detect touching by the user.

Preferably, width of the gap, in other words the distance of the film (120) from the touch surface is maintained constant, independent of whether the film (120) is stationary or being conveyed. A touch of a user, applying a force against the film (120), causes the film (120) to touch the touch surface such that the touch can be detected by the touch screen unit.

Wanted distance of the film from the touch surface (115) is preferably achieved by assembling a pair of gap determining deflector rolls (130') with predetermined diameter at predetermined positions on the two pairs of deflector roll supports (141a, 141b; 141c, 141d) attached to the touch screen unit (110). As understood by a skilled person, any orientation of the touch surface is applicable. In a typical use case, the touch screen unit is installed such that the touch surface is in approximately vertical position. The film (120) may be configured to be conveyed in vertical direction or in horizontal direction. When the film (120) is conveyed vertically in front of a vertically oriented touch surface (115), the gap determining deflector rolls (130') are disposed on the top and bottom sides of the touch screen unit (110). When the film (120) is conveyed horizontally in front of a vertically oriented touch surface (115), the gap determining deflector rolls (130') are disposed on left and right sides of the touch screen unit (110). If the touch surface (115) were positioned horizontally, the film (120) is conveyed horizontally from one side to the other of the touch screen unit (110).

The film (120) may be any suitable transparent film known in the art, such as FEP (perfluoro ethylene/propylene copolymer, tetrafluoroethylene/ hexafluoropropylene) film, PFA (perfluoroalkoxy alkane) film or ETFE (ethylene/tetrafluoroethylene) film. FEP film has been preferred in test use, as it seems to provide best transparency among these alternatives, which is an important characteristic when the film covers the touch screen. Tests have shown that thickness of the film is preferably from 0.2 mm to 0.3 mm. A thinner film increases risk of stretching, folding, punching and/or breaking the film during use. A film thinner than 0.2 mm may also cause problems in tensioning of the film, since it is easily stretched due to tension and heat produced by UVC light source used for sanitizing. A thin film is also weaker and thus more susceptible to mischief, which is a real threat for example when the apparatus is designed for public use in public spaces. On the other hand, a film thicker than 0.3 mm has been found to deteriorate touch sensitivity and thus operability of the touch screen. This may be for example because the film is too stiff, which increases the force needed to touch the surface of the touch screen by a user. Furthermore, increased thickness of the film deteriorates effectivity of the UVC sanitizing operation which will be discussed later.

Figure 5A also shows an exemplary placement of a sanitizing unit (320). The sanitizing unit should be inside the housing, on the hindside of the touch screen unit (110) with free line of sight towards the film (120). The sanitizing unit (320) may be attached to the touch screen unit (110), one or more deflector roll supports (141) or to the housing. Basically, any location of the sanitizing unit (320) within the housing is applicable, where the sanitizing unit (320) is capable of exposing the film (120), but no user is exposed to the UVC light at any time, when the touch screen unit (110) is properly assembled between the housing and the frame.

The sanitizing apparatus comprises a control apparatus (not shown) configured to control the film conveyor apparatus, in particular the drive means (350) thereof to move a sanitized portion of the film (120) in front of the touch surface (115) between user input with the touch screen, and to simultaneously move a used portion of the film (120) inside the housing for cleaning and sanitizing.

The Figure 5B shows a portion of the bearing housing means (143) to which the deflector rolls (130, 130') are attached by bearings (180). The deflector roll support is designed such that brackets (142) thereof can be easily fitted and fixed to the touch display unit (110) by fastening means. Position, as determined by design of the bracket (142) and the bearing housing means (143), in particular position of the installation hole in which the gap determining deflector roll (130') is assembled in the bearing housing means (143), and diameter of the gap determining deflector roll (130') determine distance between the touch surface (115) and the portion of the film (120) in front of it.

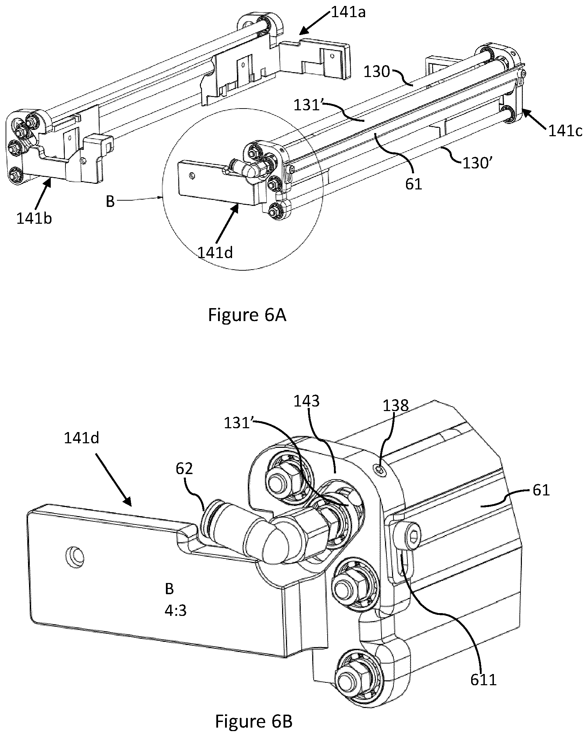

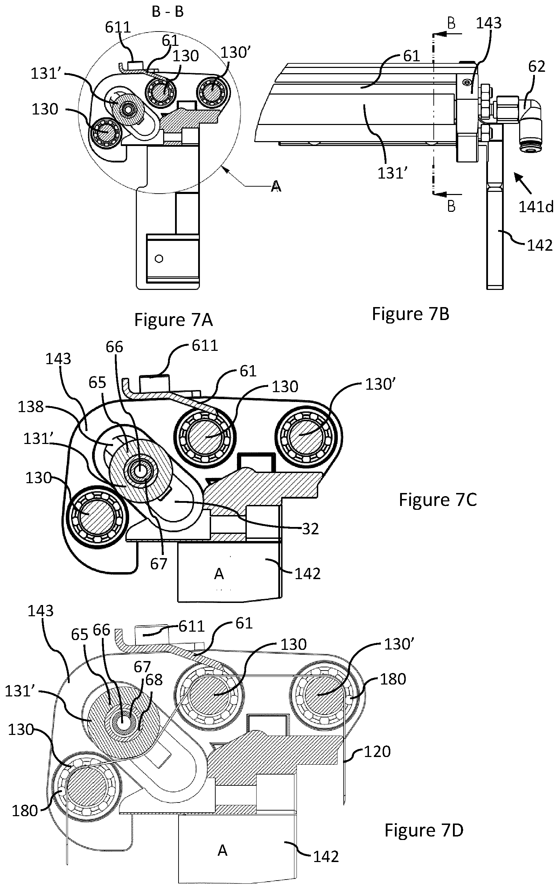

Figures 6A and 6B as well as figures 7A to 7D illustrate deflector roll supports (141) and rolls (130, 130', 131, 131', 150) of the film conveying apparatus, further equipped with optional pre-cleaning means. The film, the touch screen unit, and the drive means are omitted to give a clear view of the structural parts of deflector roll supports (141), deflector rolls and pre-cleaning means. Figure 6B is an enlarged view of portion B encircled in the cross-section figure 6A. Figures 7A and 7C illustrate a cross-section B-B of the portion of assembly shown in the figure 7B. Figure 7C is an enlarged view of portion A encircled in the figure 7A. Figure 7D illustrates the same enlarged cross-section portion A as the figure 7C, additionally showing route of the film (120) as guided by deflector rolls (130, 130', 131').

Pre-cleaning means may be implemented as part of the sanitizing unit, a separate apparatus or, as in this example, pre-cleaning means may be comprised in the film conveying apparatus. The pre-cleaning means is preferably located upstream from the sanitizing unit in view of the direction of conveying the film, such that pre-cleaning is performed before sanitizing the film with UVC light. In the example shown in the figures 6A, 6B and 7A to 7D, the pre-cleaning means is implemented in association with deflector roll arrangement that is disposed at one side of the touch screen unit (110). Pre-cleaning is preferably performed such that the sanitizing unit (320) is downstream from the pre-cleaning means in view of the conveying direction of the film (120).

The pre-cleaning means may comprise a pre-cleaning scraper (61). The pre-cleaning scraper (61) is configured to drag off any bigger scrap particles attached to the film (121). The pre-cleaning scraper (61) may be formed as at least one thin scraper leaf that extends over the entire width of the film (121). The pre-cleaning scraper (61) is advantageously removably and adjustably fixed to bearing housing means (143) at its both sides by fixing means (611) so that the pre-cleaning scraper (61) extends between a pair of deflector roll supports (141c, 141d). Cleaning action of the pre-cleaning scraper (61) can be enhanced by placing it such that the film is conveyed between the outer edge of the pre-cleaning scraper (61) and one of the deflector rolls (130). The pre-cleaning scraper (61) may be made for example of metal, such as stainless steel or aluminum alloy. If aluminum alloy is used, it is preferably selected among alloys made for use in contact with seawater, thus less susceptible to corrosion due to being possibly exposed to both dirt and pre-cleaning substances, which will be discussed later. Thickness of a metal sheet forming the body of the pre-cleaning scraper (61) is preferably about 2 to 3 mm. The straight edge of the pre-cleaning scraper (61) that is most proximate to the film (120) is preferably sharpened and disposed as close to surface of the film (120) as possible, but preferably not touching the film (120) to avoid scratching thereof.

Pre-cleaning means may comprise a wet cleaning roll (131'). In the example illustrated in the figures 6A and 6B as well as in figures 7A to 7D, one of the adjustable deflector rolls (131) has a double role, acting also as a wet cleaning roll (131').

The wet cleaning is preferably performed as another pre-cleaning step after removing bigger particles with the pre-cleaning scraper (61). The wet cleaning roll (131') may have a multi-layered design. The wet cleaning roll (131') is preferably coated with felt (65) as it's outermost layer. The felt (65) is configured to be wetted during the cleaning operation with a wet cleaning substance such as any suitable mixture of alcohol and water. Examples of suitable alcohol and water mixtures to be used as wet cleaning substance are 60% to 95% isopropanol or 60% to 90% n-propanol, wherein the percentage represents share of alcohol in the mixture. Suitability of the wet cleaning substance is determined based on compatibility with the selected film material as well as effectivity on removal of dirt expected to appear in the film, such as scrap particles, as well as grease, sweat and other chemical substances present in fingerprints. Further examples of chemical substances that may need to be removed by the wet cleaning are for example various hand sanitizers and hand lotions. Wetting the felt can be controlled by the control unit. Preferably, wetting operation is intermittent like wet cleaning process.

The cleaner the alcohol-water mixture used as wet cleaning substance is, the better it is absorbed by the felt (65) coating of the wet cleaning roll (131'). Clean alcohol-water mixture avoids the felt from being chocked or blocked up over time and avoids leaving any sticky residue on the film. Alcohols help effectively removing typical substance residues originating from various hand sanitizers used by users before using the touch screen apparatus. Alcohols also evaporate quickly, so that sanitized portion of the film does not feel moist when touched by hands of a user when the sanitized film is returned into use in front of the touch screen.

It is not necessary to use an adjustable deflector roll (131) for wet cleaning, but any available deflector roll may be used if wet cleaning is to be implemented. However, using a gap determining deflector roll (130') for wet cleaning is not recommended, since thickness of the felt (65) may vary such that diameter of the wet cleaning roll may slightly vary, which would make maintaining the wanted, very precise and narrow gap between the film (120) and the touch surface (150) difficult, if not impossible.

The wet cleaning substance may be pumped to the felt (65) of the wet cleaning roll (131') from a reservoir during the wet cleaning action, which is preferably performed while the film (120) is being moved/conveyed. The wet cleaning substance may at least partially evaporate during and/or after the wet cleaning phase and further during the sanitizing phase as well as between the phases, such that no residue remains. Additionally or alternatively, any excess wet cleaning substance may be collected and either discarded or in some cases reused, in other words returned to the wet cleaning process. Possibility to reuse wet cleaning substance reduces need to refill the reservoir and thus facilitates extending time period between maintenance operations but causes the wet cleaning substance to become contaminated over time. Figures 6A, 6B and 7B also show a pipeline (62) providing a fluid connection toward the reservoir (not shown), arranged to feed wet cleaning substance into the wet cleaning roll (131'). According to this exemplary embodiment, the wet cleaning substance is pumped or otherwise fed by the pipeline into the wet cleaning roll (131'). The wet cleaning roll (131') preferably comprises a pipe or cavity as well as means for wetting the felt (65) with the wet cleaning substance.

For example, the pipe or cavity (66) inside the wet cleaning roll has perforated walls (67, 68). According to the embodiment shown in the figure 7D, the wet cleaning roll comprises two nested, perforated tubes. The inner tube (67) that is in fluid connection with the input pipeline (62) is less densely perforated, while the outer tube (68) adjacent to the felt (65) is more densely perforated. This arrangement facilitates even distribution of the wet cleaning substance into the felt (65). Because the inner tube (67) has few perforations, the wet cleaning substance flows over the entire length of the inner tube (67). This ensures that wet cleaning substance is delivered over the entire operation length of the wet cleaning roll (131'). The wet cleaning substance running through the perforations in the inner tube (67) is then spread evenly to the felt (65) by the dense perforation. Thickness of the felt (65) may be for example 2 mm.

To further improve the wet cleaning step, the wet cleaning roll (131') may be configured to rotate in opposite direction in comparison to the simultaneous movement of the film. This enables the wet cleaning roll (131') to effectively wash away dirt from the film, by enhanced scrubbing action by the increased relative motion of the felt and the film. Preferably the wet cleaning roll (131') is operated only when the film is being moved. According to some embodiments, the rotation of the wet cleaning roll is directly or indirectly caused by the same drive means (350) that rotates the drive roll (150). This can be implemented for example by a suitable drive belt arrangement or like. Alternatively, a separate drive means may be provided for rotating the wet cleaning roll (131'). Operation of the separate drive means is preferably controlled by the control unit.

Since the wet cleaning roll (131') does not require as accurate rotation drive as for example the drive roll (150), rotation of the wet cleaning roll may be arranged indirectly. For example, one of the deflector rolls (130) located in proximity of the wet cleaning roll (131') may be mechanically coupled to the wet cleaning roll (131') using toothed belt such that it causes the wet cleaning roll (131') to rotate in opposite direction in comparison to conveying direction of the film (120). To further improve this indirect drive action, the deflector roll (130) used for driving the wet cleaning roll (131') is preferably coated with friction increasing coating, which prevents slipping of the deflector roll (130) with respect to the film (120). No dedicated driving arrangement, such as operational coupling to the drive roll by a drive belt, is necessary for the deflector roll (130), but sufficient force to rotate the wet cleaning roll may be indirectly provided by movement of the film (120) against the deflector roll (130) coated with friction increasing coating.

Preferably, only after pre-cleaning the film with the pre-cleaning scraper (61) and/or the wet cleaning roll (131'), the film is sanitized using short-wave ultraviolet radiation also known as ultraviolet C radiation or UVC light. UVC sanitizing may also be characterized as disinfection. UVC light is known to be effective in killing microbes, including most known bacteria and viruses. Applying UVC radiation on the thin, transparent film, microbes are killed effectively from both sides of the film, as well as from any mechanical elements inside the housing that are exposed to the UVC light. Thus, the UVC light source may be located on either side of the film (120). Using FEP or PFA film is particularly beneficial, since these are both known to efficiently pass UVC light. UVC light exposure can occur both during movement of the film and during periods of non-movement of the film. If longer periods of UVC light exposure time are preferred, it is preferable to expose the portion of the film that is being sanitized during periods when the film does not move. This way the time of exposure can be more precisely controlled by the control unit, which ensures high level of sanitizing.

The control unit preferably is communicatively connected to at least one sensor device, which enables detecting presence of a user in vicinity of the touch screen. According to some embodiments, the touch screen unit comprises one or more sensor device for detecting presence of a user in vicinity of the touch screen and provides a signal to the control unit indicating presence of a (potential) user. The sensor could be for example any proximity sensor known in the art.

The control unit controls the film conveyor apparatus, in particular the drive means thereof, such that the film is not conveyed when the control unit determines, based on information received from the one or more sensors and/or from the touch display unit that there is a user in proximity of the touch screen, thus using or being likely to use the touch screen. Further, the control unit controls pre-cleaning and sanitizing steps, such as activation of the UVC light source and, when applicable, pumping the wet cleaning substance and rotating the wet cleaning roll. Upon detecting that the user has left the proximity of the touch screen, the control unit causes the film conveyor apparatus to convey the film such that a previously sanitized, clean portion of the film is brought in front of the touch surface while the used portion of the film is moved inside the apparatus for pre-cleaning and sanitizing. Thus, a new user arriving at the touch screen will always have a clean and microbiologically safe surface available for use. Furthermore, the touch screen unit may provide indication of detected use of the touch screen as additional information available for the control unit. Based on said detected use information, the control unit may further determine whether the touch screen was actually used during detected proximity of the user. This enables suppressing conveying of the film if it was not touched even though there was a person detected in the proximity of the touch screen, which saves energy, resources such as cleaning substance as well as reduces wear of the film and the film conveying apparatus itself.

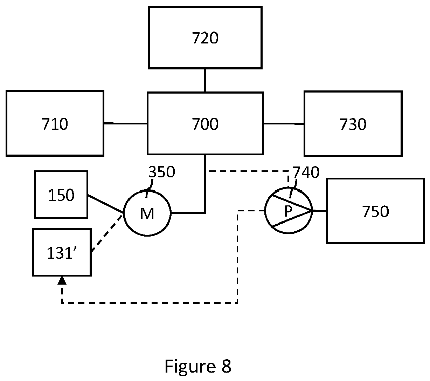

The figure 8 illustrates schematically control arrangement of the film sanitizing apparatus. A controller (700) is communicatively coupled to one or more sensors (710) and/or to a controller (720) the touch screen unit (110) coupled to one or more sensors (710) for receiving information on proximity of a user and/or use of the touch screen. The controller (700) is communicatively coupled to at least one memory (730) for storing operation instructions and data. The controller controls operation of the film conveying apparatus by controlling the drive means (350), such as motor, that operates the drive roll (150). If wet cleaning is implemented, the controller may be communicatively coupled to a pump (740) for controlling supplying of the wet cleaning substance, and if the wet cleaning roll (131') is arranged to rotate, the controller further controls the drive means (350) or another drive means (not shown) that is arranged to rotate the wet cleaning roll (131'). Under control of the control unit, the pump (740) pumps wet cleaning substance from a reservoir (750) to the wet cleaning roll (131').

It is apparent to a person skilled in the art that as technology advanced, the basic idea of the invention can be implemented in various ways. The invention and its embodiments are therefore not restricted to the above examples, but they may vary within the scope of the claims.

## Claims

1. A sanitizing apparatus for providing a sanitized user interface for a touch screen unit (110), the sanitizing apparatus comprising a film conveyor apparatus, a housing, a film (120), a control unit and a sanitizing unit (320), the film conveyor apparatus is configured to move a sanitized portion of the film in front of a touch surface (115) of the touch screen unit (110), and the sanitizing apparatus comprises a sanitizing unit (320) configured to sanitize a portion of the film (120) by means of UVC light after the portion of the film (120) has been used for user interaction, wherein the portion of the film (120) to be sanitized is enclosed inside the housing during said sanitization, and wherein the film conveyor apparatus comprises:
- a chassis, and
- a plurality of rolls (130, 130', 131, 150) attached to the chassis, the plurality of rolls comprising a plurality of deflector rolls (130, 130', 131) configured to guide the film (120) about the touch screen unit (110), and a drive roll (150) configured to drive the film (120),
**characterized in that** the plurality of rolls is arranged in two groups of rolls (130, 130', 131, 150), wherein the chassis comprises two pairs of deflector roll support means (141) configured to be assembled on two opposite sides of the touch screen unit (110) by attaching the deflector roll support means (141) to the touch screen unit (110), wherein each pair of deflector roll support means (141) is configured to rotatably support all rolls of one of the two groups of rolls.

2. The sanitizing apparatus according to claim 1, wherein each deflector roll support means (141) comprises
- a bracket (142) configured for fitting and removably attaching the deflector roll support means (141) to a desired position of the touch screen unit (110), and
- a bearing housing means (143) configured to house bearings that enable rotation of rolls (130, 130', 131, 150) and to determine position of rolls (130, 130', 131, 150) supported thereby with respect to the touch screen unit (110).

3. The sanitizing apparatus according to claim 2, wherein bearing housing means (143) of each pair of deflector support means are mutually parallel and installation holes configured to receive bearings and axes of rolls (130, 130', 131, 150) in the two parallel bearing housing means are mutually mirrored.

4. The sanitizing apparatus according to claim 2 or 3, wherein at least two of the bearing housing means (143) are configured to be slidably coupled to the respective bracket (142), wherein the bearing housing means (143) is configured to be slidable with respect to the respective bracket (142) only in direction parallel to the longitudinal axis of rolls (130, 130', 131', 150) supported thereby, and wherein the bearing housing means (143) is configured to be removably fixed to the bearing housing means (143) by means of coupling of the rolls (130, 130', 131', 150) to the bearing housing means (143).

5. The sanitizing apparatus according to any one of claims 1 to 4, wherein one deflector roll in both groups of rolls is configured to operate as gap determining deflector roll (130'), wherein these two gap determining deflector rolls (130') are disposed on two opposite sides of the touch surface (115) of the touch screen device (110), and wherein said gap determining deflector rolls (130') are configured to determine and maintain a predetermined distance of the portion of the film (120) in front of the touch surface (115) when the touch screen (115) is not used for user input during a user interaction.

6. The sanitizing apparatus according to any one of claims 1 to 5, wherein bearing housing means (143) of each pair of deflector roll support means (141) comprise mutually mirrored arrangement of installation holes configured to house bearings (180) and rolls (130, 130', 131, 150).

7. The sanitizing apparatus according to any one of claims 1 to 6, wherein at least one of the deflector rolls is an adjustable deflector roll (131), wherein the position of the adjustable deflector roll (131) is configured to adjust tension of the film (120).

8. The sanitizing apparatus according to any one of claims 1 to 7, wherein outer surface of the drive roll (150) is coated with a friction increasing coating.

9. The sanitizing apparatus according to claim 8, wherein outer surface of a deflector roll (130, 131) is coated with a friction increasing coating, and wherein the deflector roll coated with the friction increasing coating is operationally coupled with the drive roll (150) for applying drive action on the film (120) at both sides of the touch screen unit (110).

10. The sanitizing apparatus according to any one of claims 1 to 9, wherein the sanitizing apparatus comprises at least one of a pre-cleaning scraper (61) and a wet cleaning roll (131'), wherein the pre-cleaning scraper (61) is configured to drag off scrap particles from the film (120), wherein outer surface of the wet cleaning roll (131') is covered by a felt (65), wherein the felt (65) is configured to be wetted with a wet cleaning substance, and wherein the wet cleaning roll (131') is configured to wash away scrap particles, grease and various chemical substances from the film (120).

11. The sanitizing apparatus according to claim 10, when depending on claim 7, wherein the at least one adjustable deflector roll (131) is further configured to operate as the wet cleaning roll (131').

12. The sanitizing apparatus according to claim 10 or 11, wherein the wet cleaning roll (131') is configured to rotate in opposite direction in comparison to the movement of the film (120).

13. The sanitizing apparatus according to claim 12, wherein outer surface of a deflector roll (130) is coated with a friction increasing coating, and wherein the deflector roll coated with the friction increasing coating is operationally coupled with the wet cleaning roll (131') for rotating the wet cleaning roll.

14. The sanitizing apparatus according to any one of claims 10 to 13, wherein the film sanitizing unit is configured to expose the film (120) to UVC light after pre-cleaning step performed by at least one of the pre-cleaning scraper (61) and the wet cleaning roll (131').

15. A touch screen apparatus comprising a touch screen unit (110) and the sanitizing apparatus according to any one of claims 1 to 14.
